# EUROPEAN PATENT SPECIFICATION

(11) **EP 3 815 714 B1**
(45) Date of publication and mention of the grant of the patent: **31.07.2024**
(21) Application number: 19824518.5
(22) Date of filing: 24.06.2019
(51) Int. Cl.: A61K 47/04, A61K 9/14, A61K 9/30, A61K 9/36, A61K 47/14, A61K 47/26, A61K 47/32

(54) **COATING COMPOSITION, ORAL SOLID PREPARATION AND METHOD FOR PRODUCING SAME**
BESCHICHTUNGSZUSAMMENSETZUNG, ORALE FESTE ZUBEREITUNG UND VERFAHREN ZU IHRER HERSTELLUNG
COMPOSITION D'ENROBAGE AINSI QUE PRÉPARATION SOLIDE DESTINÉE À ÊTRE ADMINISTRÉE PAR VOIE ORALE ET SON PROCÉDÉ DE FABRICATION

(30) Priority: 25.06.2018 JP 2018119467
(43) Date of publication of application: 05.05.2021
(73) Proprietor: Japan Vam & Poval Co., Ltd., Sakai-shi, Osaka 592-8331 (JP)
(72) Inventor: KAWADA, Shotaro, Sakai-shi, Osaka 592-8331 (JP); KAWANISHI, Masatoshi, Sakai-shi, Osaka 592-8331 (JP)
(74) Representative: Kraus & Lederer PartGmbB
(86) International application number: PCT/JP2019/024874
(87) International publication number: WO 2020/004297

(56) References cited:
- JP-A- 2003 509 339
- JP-A- 2010 189 378
- JP-A- 2011 207 873
- JP-A- 2016 196 438
- JP-A- 2016 196 439
- JP-A- 2017 101 021
- US-A1- 2017 348 243
- US-A1- 2017 354 608
- MANABU SENOO, KAORU TSUJII: "Surfactant Chemistry and Applications; 1st ed.", 25 August 2003, DA INIPPON TOSHO CO, ISBN: 4-477-00509-1, article "Passage; Surfactant Chemistry and Applications", pages: 24, 51, XP009524605
- JAPAN OIL CHEMISTRY ASSOCIATION: "Oleochemistry Handbook, Revised 3rd edition", 28 February 1990, MARUZEN, ISBN: 4-621-03447-2, article "Passage, Oleochemistry Handbook", pages: 490 - 491, XP009524602
- KAWADA, SHOTARO: "New materials 8 that contribute to formulation design, Moisture-proof coating using PVA", PHARM TECH JAPAN, vol. 35, no. 1, January 2019 (2019-01-01), pages 31 - 33, XP009524699, ISSN: 0910-4739

## Description

### TECHNICAL FIELD

The present invention relates to a coating composition used for oral solid preparations, an oral solid preparation coated with the coating composition, and a method for producing the oral solid preparation, etc.

### BACKGROUND ART

Film coating and sugar coating are widely used techniques for coating of oral solid preparations such as drug-containing solid preparations (e.g., tablets etc.) for the purpose of masking of drug-related unpleasant taste, oxygen insulation, moisture-proofing, product appearance improvement, etc.

Film coating is more useful than sugar-coating because it can be performed in a faster and simpler way and can provide a thinner coating film to allow the production of smaller solid preparations, which are easily ingestible oral solid preparations.

For film coating, various polymers including hydroxypropylmethylcellulose (hereinafter abbreviated as HPMC) are used as base materials, and recently, polyvinyl alcohol (hereinafter may be abbreviated as PVA) has drawn attention.

PVA films are excellent in moisture-proofness, and PVA film coating improves the storage stability of solid preparations containing highly hygroscopic components.

However, when PVA is used as a base material of coating compositions, a highly tacky aqueous PVA solution is likely to cause adhesion between solid preparations, adhesion of solid preparations on the inner surface of a coating apparatus, and other undesired events during coating, which hinder the increase of the spray rate, leading to low productivity.

A known solution to the above problem is to use a coating composition containing PVA in combination with an additive capable of reducing the tackiness of PVA in coating of solid preparations, thereby reducing the adhesion between solid preparations during coating.

For example, Patent Literature 1 discloses a coating composition comprising PVA in combination with a plasticizer and talc. Patent Literature 1 states that the combination of PVA with a plasticizer and talc reduces the tackiness of PVA, resulting in highly productive coating providing high-quality appearance.

However, the coating composition of Patent Literature 1 has a problem in that the addition of a plasticizer such as polyethylene glycol reduces the tackiness of PVA but deteriorates the distinctive moisture-proof performance of

PVA.

### CITATION LIST

### Patent Literature

Patent Literature 1: JP-W 2003-509339.

US-2017348243 relates to a coating composition comprising polyvinyl alcohol (PVA), a sugar, a plasticizer or combination of plasticizers, and other pharmaceutically acceptable excipients.

### SUMMARY OF INVENTION

### TECHNICAL PROBLEM

An object of the present invention is to provide a novel coating composition for oral solid preparations.

Another object of the present invention is to provide a coating composition for oral solid preparations which coating composition forms a highly moisture-proof coating film.

Another object of the present invention is to provide a coating composition for oral solid preparations which coating composition is less likely to develop tackiness during coating.

Another object of the present invention is to provide a coating composition for oral solid preparations which coating composition forms a coating film having excellent surface smoothness.

Another object of the present invention is to provide a coating composition for oral solid preparations which coating composition is practical and applicable to common oral solid preparations.

Yet another object of the present invention is to provide an oral solid preparation coated with the above coating composition.

Yet another object of the present invention is to provide a method for producing an oral solid preparation coated with the above coating composition.

### SOLUTION TO PROBLEM

The present inventors conducted extensive research to achieve the above-mentioned objects. As a result, the present inventors found that a coating composition comprising a PVA polymer (a), a fatty acid ester of a polyol having 3 or more hydroxy groups which meets specific requirements (b), and a specific percentage of talc (c) is less likely to develop tackiness during coating and can provide highly productive coating of solid preparation. The scope of the invention is defined by the claims.

That is, the present invention relates to the following.
[1] A coating composition for oral solid preparations, the coating composition comprising a polyvinyl alcohol-based polymer (a), a fatty acid ester of a polyol having 3 or more hydroxy groups (b), and talc (c),
   wherein the coating composition meets the following requirements (1) to (4):
   (1) the fatty acid ester of a polyol having 3 or more hydroxy groups (b) has a hydrophilic lipophilic balance (HLB) value of 4.0 or more;
   (2) the fatty acid ester of a polyol having 3 or more hydroxy groups (b) has 12 to 22 carbon atoms per fatty acid ester unit; and
   (3) the talc (c) is present at 50% by mass or less of the whole composition, and
   (4) the proportion of the polyol fatty acid ester (b) in the whole composition is 5 to 30% by mass.
[2] The coating composition for oral solid preparations according to the above [1], wherein the fatty acid ester of a polyol having 3 or more hydroxy groups (b) comprises at least one kind selected from a sugar fatty acid ester and a sugar alcohol fatty acid ester.
[3] The coating composition for oral solid preparations according to the above [1], wherein the fatty acid ester of a polyol having 3 or more hydroxy groups (b) comprises a sorbitan fatty acid ester.
[4] The coating composition for oral solid preparations according to any one of the above [1] to [3], wherein the polyvinyl alcohol-based polymer (a) and the fatty acid ester of a polyol having 3 or more hydroxy groups (b) are present at a mass ratio of 95:5 to 50:50 in the coating composition.
[5] The coating composition for oral solid preparations according to any one of the above [1] to [4], the coating composition further comprising a colorant.
[6] The coating composition for oral solid preparations according to any one of the above [1] to [5], wherein a 4% by mass aqueous solution of the polyvinyl alcohol-based polymer (a) has a viscosity of 2.0 to 10.0 mPa·s.
[7] An oral solid preparation coated with the coating composition according to any one of the above [1] to [6].
[8] A method for producing an oral solid preparation, the method comprising the step of applying or spraying an aqueous and/or water-based solution containing the coating composition according to any one of the above [1] to [6] to a solid preparation to cover a surface of the solid preparation with the coating composition.

### ADVANTAGEOUS EFFECTS OF INVENTION

The present invention provides a novel coating composition for oral solid preparations.

The coating composition comprises a PVA polymer (a), a fatty acid ester of a polyol having 3 or more hydroxy groups which meets specific requirements (b), a specific percentage of talc (c) and a specific proportion of the polyol fatty acid ester in the whole composition forms a coating film having high moisture-proofness (particularly, high moisture-proofness even under humid conditions) .

When the coating composition of the present invention is used for coating of solid preparations, adhesion between solid preparations is less likely to occur, leading to reduction in coating time and improvement in coating productivity.

The coating composition of the present invention can provide oral solid preparations with excellent surface smoothness.

The coating composition of the present invention is practical and applicable to common oral solid preparations.

In addition, the present invention provides an oral solid preparation coated with the above coating composition.

Further, the present invention provides a method for producing an oral solid preparation coated with the above coating composition. In this method, less adhesion between solid preparations during coating results in efficient coating.

### DESCRIPTION OF EMBODIMENTS

### Coating composition for oral solid preparations

The coating composition of the present invention for oral solid preparations comprises a polyvinyl alcohol-based polymer (a), a fatty acid ester of a polyol having 3 or more hydroxy groups (b), and a specific amount of talc (c). In the composition, the fatty acid ester of a polyol having 3 or more hydroxy groups (b) meets specific requirements as described later.

The components of the coating composition of the present invention usually refer to the components other than a solvent (e.g., a liquid component such as water or an organic solvent) in a coating composition solution used in coating. When the coating composition is described below, the mass ratio and mass percentage of each component in the coating composition usually refer to a proportion relative to the components other than a solvent.

### Polyvinyl alcohol-based polymer (a)

The polyvinyl alcohol-based polymer (may be referred to as a PVA-based polymer, PVA, etc.) (a) is usually a saponified product of a vinyl ester-based polymer (a polymer composed of a vinyl ester as at least a polymerization component).

The vinyl ester (vinyl ester monomer) is not particularly limited, and examples include fatty acid vinyl esters [e.g., C₁₋₂₀ fatty acid vinyl esters (e.g., C₁₋₁₆ alkanoic acid vinyl esters) such as vinyl formate, vinyl acetate, vinyl propionate, vinyl butyrate, vinyl caprylate, vinyl versatate, and vinyl monochloroacetate], and aromatic carboxylic acid vinyl esters [e.g., vinyl arenecarboxylates (e.g., C₇₋₁₂ arene carboxylic acid vinyl esters) such as vinyl benzoate] .

One kind of vinyl ester or a combination of two or more kinds of vinyl esters may be used.

The vinyl ester preferably at least contains a fatty acid vinyl ester (e.g., C₁₋₁₀ alkanoic acid vinyl esters etc., such as vinyl formate, vinyl acetate, vinyl propionate, and vinyl butyrate). Particularly, vinyl acetate is preferred from industrial or other viewpoints.

The vinyl ester-based polymer has a vinyl ester unit, and if necessary, may have an additional monomer unit (a monomer capable of copolymerizing with vinyl esters) (in other words, the vinyl ester-based polymer may be modified with an additional monomer).

The additional monomer is not particularly limited, and examples include, but are not limited to, alpha-olefins (e.g., ethylene, propylene, etc.), (meth)acrylic acid esters [e.g., (meth) acrylic acid alkyl esters such as methyl (meth) acrylate, ethyl (meth)acrylate, butyl (meth)acrylate, and 2-ethylhexyl (meth)acrylate], unsaturated amides [e.g., (meth)acrylamide, diacetone acrylamide, N-methylolacrylamide, etc.], unsaturated acids {e.g., unsaturated acids [e.g., (meth) acrylic acid, crotonic acid, maleic acid, itaconic acid, fumaric acid, etc.], unsaturated acid esters [unsaturated acid esters other than (meth)acrylic acid esters, e.g., alkyl (methyl, ethyl, propyl, etc.) esters etc.], unsaturated acid anhydrides (maleic anhydride etc.), salts of unsaturated acids [e.g., alkali metal salts (e.g., sodium salts, potassium salts, etc.), ammonium salts, etc.], etc.}, glycidyl group-containing monomers [e.g., allyl glycidyl ethers, glycidyl (meth)acrylate, etc.], sulfonic group-containing monomers (e.g., 2-acrylamide-2-methylpropane sulfonic acid, salts thereof, etc.), phosphate group-containing monomers [e.g., acid phosphoxy ethyl (meth)acrylate, acid phosphoxy propyl (meth) acrylate, etc.], vinyl ethers (e.g., alkyl vinyl ethers), allyl alcohols, etc.

One kind of additional monomer or a combination of two or more kinds of additional monomers may be used.

In the PVA-based polymer (a), some vinyl alcohol units may be modified by a reaction, such as acetalization, etherification, acetoacetylization, or cationization.

One kind of PVA-based polymer (a) or a combination of two or more kinds of PVA-based polymers (a) may be used.

The PVA-based polymer (a) may be a commercial product.

The method for producing the PVA-based polymer (a) is not particularly limited, and known methods, for example, saponification of a vinyl ester-based polymer, are may be used.

The polymerization method for the vinyl ester-based polymer is not particularly limited, and examples include known polymerization methods such as block polymerization, solution polymerization, suspension polymerization, and emulsion polymerization. Among them, solution polymerization (e.g., solution polymerization using methanol as a solvent) is industrially preferred.

In the solution polymerization, known initiators such as peroxide initiators and azo initiators can be used, and the polymerization degree of the vinyl ester-based polymer can be adjusted by varying the feed ratio of the vinyl ester monomer and a solvent and the polymerization yield.

The method for saponifying the vinyl ester-based polymer can be a conventional saponification method using an alkaline or acid catalyst. In particular, industrially preferred is alcoholysis, which is performed by adding an alkali such as sodium hydroxide to a solution of the vinyl ester-based polymer in methanol or in a mixed solvent of methanol, water, methyl acetate, etc. and stirring the mixture.

After that, optionally, the obtained mass product, gelled product, or granular product is pulverized, and if needed, the alkali is neutralized; then the solid matter is separated from the liquid matter and dried to yield a PVA-based polymer.

The saponification value of the PVA-based polymer (a) is not particularly limited and is preferably within the standard of the saponification value of PVA described in the following three official compendiums: the Japan Pharmaceutical Excipient Standards, the United States Pharmacopeia, and the European Pharmacopoeia. In addition, the average saponification value of the PVA-based polymer is, for example, preferably 74.0 mol% to 89.0 mol% (e.g., 80.0 to 89.0 mol% etc.) and particularly preferably 85.0 mol % to 89.0 mol% for fast dissolution in a living body.

When the average saponification value is 85.0 mol% or more, the PVA-based polymer (a) can be used as a material of pharmaceutical preparations adapted for global markets. In addition, the proportion of hydrophobic groups in such a PVA-based polymer (a) is low enough so that the PVA-based polymer (a) is highly hydrophilic, less likely to precipitate at high temperature in preparing an aqueous solution, and easy to handle. That is why the lower limit specified above is particularly preferred.

Similarly, when the average saponification value is 89.0 mol% or less, the PVA-based polymer (a) can be used as a material of pharmaceutical preparations adapted for global markets. In addition, the proportion of hydroxyl groups in PVA is not so high as to result in reduced water solubility due to high crystallinity or reduced dissolution rate of oral solid preparations coated with the coating composition of the present invention. That is why the upper limit specified above is particularly preferred.

The method for measuring the average saponification value of PVA is not particularly limited, and for example, the method for measuring the saponification value specified in JIS K6726 can be used.

The polymerization degree of the PVA-based polymer (a) is not particularly limited, and the viscosity of its 4% by mass aqueous solution is, for example, preferably 2.0 to 10.0 mPa·s (e.g., 3.0 to 9.0 mPa·s etc.) and more preferably 3.0 to 7.0 mPa·s.

When the viscosity of the 4% by mass aqueous solution is 2.0 mPa·s or more, a high-strength coat layer can be formed on the surface of solid preparations after coating. That is why the lower limit specified above is preferred. When the viscosity of the 4% by mass aqueous solution is 10 mPa·s or less, the viscosity is low enough to allow a high-rate spray during coating, resulting in high productivity. That is why the upper limit specified above is preferred.

The method for measuring the viscosity of the 4% by mass aqueous solution is not particularly limited, and for example, the method specified in JIS K6726 can be used for viscosity measurement.

### Fatty acid ester of a polyol having 3 or more hydroxy groups (b)

In the fatty acid ester of a polyol having 3 or more hydroxy groups (b) (hereinafter may be referred to simply as a polyol fatty acid ester (b)), the number of hydroxy groups is 3 or more, and for example, but not particularly limited to, 3 to 10 (e.g., 3 to 8 etc.).

Examples of the polyol having 3 or more hydroxy groups include sugars [e.g., monosaccharides (e.g., glucose, galactose, mannose, fructose, etc.), disaccharides (e.g., saccharose, maltose, lactose, trehalose, etc.)], sugar alcohols (e.g., erythritol, lactitol, maltitol, mannitol, sorbitol, xylitol, inositol, sorbitan, etc.), alkane polyols [e.g., alkane triols (e.g., C₃₋₁₀ alkane triols, such as glycerin, butanetriol, and hexanetriol, preferably C₃₋₆ alkane triols)], polyalkane polyols {e.g., polyalkane triols [e.g., polyglycerin (e.g., diglycerin, triglycerin) and other polyalkane triols, preferably di- to tri-C₃₋₆ alkane triols]}, etc.

In addition, the polyol having 3 or more hydroxy groups may be in the form of an adduct with an oxyalkylene (e.g., a polyoxyalkylene adduct). Examples of such an adduct include polyoxyalkylene adducts of the exemplary compounds listed above [e.g., polyoxyalkylene sorbitan (e.g., polyoxyethylene sorbitan etc.), polyoxyalkylene glycerin (e.g., polyoxyethylene glycerin etc.), etc.].

Among the foregoing, sugars, sugar alcohols, etc. are preferred, and saccharose, sorbitan, etc. are particularly preferred.

One kind of polyol having 3 or more hydroxy groups or a combination of two or more kinds of polyols having 3 or more hydroxy groups may be used.

The polyol fatty acid ester (b) usually and desirably meets the requirements (1) and (2) . The meaning of these requirements is described below.
Requirement (1): the fatty acid ester of a polyol having 3 or more hydroxy groups (b) has an HLB value of 4.0 or more.

HLB is short for hydrophilic lipophilic balance, and the HLB value varies with the degree of fatty acid esterification in the molecule. A high proportion of fatty acid monoesters in the molecule corresponds to a high HLB value, indicating high hydrophilicity. On the other hand, a high proportion of polyesters such as diesters and triesters and a low proportion of monoesters in the molecule correspond to a low HLB value, indicating high hydrophobicity. In the present invention, the HLB value is preferably 4.0 or more (e.g., 4 to 20, 4 to 18, 10 to 18, 12 to 18, etc.). When the HLB value is 4.0 or more, the proportion of monoesters is high enough to make it easy to dissolve and/or disperse the coating composition in water to prepare a uniform aqueous solution. That is why the lower limit specified above is preferred.

The method for calculating the HLB value is not particularly limited, and for example, the HLB value may be calculated according to the Atlas HLB system (HLB value = 20 (1-S/A) wherein S is a saponification value and A is an acid value of a fatty acid) .

Next, requirement (2) is described.
Requirement (2) : the fatty acid ester of a polyol having 3 or more hydroxy groups (b) has 12 to 22 carbon atoms per fatty acid ester unit.

The fatty acid ester is a hydrophobic group and may be saturated or unsaturated and straight-chained or branched-chained. The hydrophobicity of the fatty acid ester varies with the number of carbon atoms.

In the present invention, the number of carbon atoms per fatty acid ester unit is 12 to 22 (e.g., 14 to 22, 12 to 20, 12 to 18, etc.). When the number of carbon atoms is 12 or more, the proportion of hydrophobic groups in the molecule is high enough to provide excellent moisture-proofing in combination with PVA. That is why the lower limit specified above is preferred. When the number of carbon atoms is 22 or less, the proportion of hydrophobic groups in the molecule is low enough to make it easy to dissolve and/or disperse the coating composition in water to prepare a uniform aqueous solution. That is why the upper limit specified above is preferred.

Examples of the fatty acid constituting the fatty acid ester unit include C₁₂₋₂₂ fatty acids {e.g., C₁₂₋₂₂ saturated fatty acids [e.g., C₁₂₋₂₂ alkanoic acids, such as lauric acid, myristic acid, pentadecyl acid, palmitic acid, heptadecanoic acid, and stearic acid], C₁₂₋₂₂ unsaturated fatty acids [e.g., C₁₂₋₂₂ mono-unsaturated fatty acids (e.g., C₁₂₋₂₂ alkenoic acids, such as oleic acid, myristoleic acid, and palmitoleic acid), C₁₂₋₂₂ di- to hexa-unsaturated fatty acids (e.g., C₁₂₋₂₂ alkadienoic acids, such as linoleic acid, C₁₂₋₂₂ alkatrienoic acids, such as linolenic acid), etc.]} etc.

One kind of fatty acid or a combination of two or more kinds of fatty acids may be used.

One kind of polyol fatty acid ester (b) or a combination of two or more kinds of polyol fatty acid esters (b) may be used.

### Talc (c)

The talc (c) is not particularly limited and is, for example, a talc generally used as an additive for pharmaceutical and food products, preferably a talc specified in the Japanese Pharmacopoeia.

In the present invention, the amount of the talc in the coating composition usually meets requirement (3) as described later.

### Additional components

The coating composition for oral solid preparations may comprise an additional component other than the polyvinyl alcohol-based polymer (a), the fatty acid ester of a polyol having 3 or more hydroxy groups (b), and the talc (c).

The additional component is not particularly limited, and examples include various additives such as colorants [pigments (e.g., titanium oxide, aluminum lake, iron oxide, etc.), natural colorants, etc.], drugs usually used for pharmaceutical preparations, lubricants (e.g., magnesium stearate, calcium stearate, stearic acid, etc.), polymers other than the PVA-based polymer (a) (e.g., hydroxypropylmethyl cellulose, hydroxypropyl cellulose, etc.), surfactants other than the polyol fatty acid ester (b), sweeteners, coating materials, defoamers, pH adjusters, etc.

Among them, colorants are preferred.

Among colorants, preferred is titanium oxide. One of the reasons is that it can provide hue improvement on the surface of solid preparations after coating.

One kind of additional component or a combination of two or more kinds of additional components may be used.

### Embodiments of coating composition for oral solid preparations

In the composition, the proportion of the PVA-based polymer (a) (the proportion of the PVA-based polymer (a) in the whole composition) is not particularly limited and may be, for example, about 25 to 90% by mass (e.g., 30 to 80% by mass), preferably about 35 to 70% by mass, and more preferably about 40 to 60% by mass.

In the composition, the proportion of the polyol fatty acid ester (b) (the proportion of the polyol fatty acid ester (b) in the whole composition) is about 5 to 30% by mass, and preferably about 5 to 20% by mass (e.g., 7 to 15% by mass).

In the composition, the proportion of the talc (c) (the proportion of the talc (c) in the whole composition) usually and desirably meets requirement (3): the talc (c) is present at 50% by mass or less of the whole composition.

In the composition, the proportion of the talc (c) is less than 50% by mass, 49% by mass or less, 45% by mass or less, 40% by mass or less, or the like.

The lower limit of the proportion of the talc (c) in the composition is not particularly specified, and the proportion of the talc (c) is, for example, preferably 5% by mass or more (e.g., 10% by mass or more, 15% by mass or more, etc.) and more preferably 30% by mass or more.

When the proportion of the talc is 5% by mass or more, a highly moisture-proof coat layer can be formed from the coating composition, and the surface smoothness after coating is excellent. That is why the lower limit specified above is preferred. When the proportion of the talc is 50% by mass or less, the coat layer after coating hardly becomes brittle . That is why the upper limit specified above is preferred.

In the composition comprising the additional component, the proportion of the additional component (the proportion of the additional component in the whole composition) is not particularly limited and may be, for example, about 1 to 50% by mass, preferably about 2 to 30% by mass, and more preferably about 5 to 20% by mass.

In the composition, the ratio of the PVA-based polymer (a) and the polyol fatty acid ester (b) is not particularly limited, and the ratio (a): (b) (mass ratio) is, for example, preferably 95:5 to 50:50, more preferably 90:10 to 60:40, and particularly preferably 80:20 to 70:30.

When the proportion of (b) is higher than 95:5 (mass ratio (a) : (b)), the tackiness of PVA is reduced enough to result in less adhesion between solid preparations during coating. That is why the lower limit specified above is preferred.

When the proportion of (b) is lower than 50:50 (mass ratio (a) : (b)), a high-strength coat layer can be formed. That is why the upper limit specified above is preferred.

In the composition, the ratio of the PVA-based polymer (a) and the talc (c) is not particularly limited, and the ratio (a) : (c) (mass ratio) may be, for example, 90:10 to 30:70, preferably 80:20 to 40:60, and more preferably 70:30 to 50:50.

In the composition comprising the additional component, the ratio of the additional component to the PVA-based polymer (a) is not particularly limited, and for example, the amount of the additional component is preferably 1 to 100 parts by mass (e.g., 1 to 80 parts by mass, 1 to 60 parts by mass, etc.) and more preferably 1 to 50 parts by mass relative to 100 parts by mass of the PVA-based polymer (a).

The form of the coating composition for oral solid preparations in use is not particularly limited, and the coating composition may be prepared in a liquid form (e.g., an aqueous solution, a water-based solution, etc.) by dissolution or dispersion in a solvent (e.g., water, an organic solvent, a mixed solvent of water and an organic solvent, etc.).

The organic solvent is not particularly limited, and examples include alcohols (e.g., ethanol etc.) etc.

One kind of solvent or a combination of two or more kinds of solvents may be used.

The solvent is preferably only water in view of environmental impact, the influence of the residual organic solvent in solid preparations, and other perspectives.

For the dissolution or dispersion of the coating composition in the solvent, known methods may be used. For example, the coating composition is dispersed in a solvent (a solvent heated if needed, e.g., water, hot water, etc.), and the mixture is stirred with heating or at ordinary temperature to prepare a coating composition solution.

Alternatively, the PVA-based polymer (a), the fatty acid ester of a polyol having 3 or more hydroxy groups (b), and the talc (c) may be separately added to solvents (solvents heated if needed, e.g., water, hot water, etc.) to prepare a coating composition solution.

In the case where the coating composition for oral solid preparations is used in a liquid form, the solid content in the liquid may be, for example, about 1 to 50% by mass and preferably about 1 to 30% by mass.

### Oral solid preparation

The present invention also includes an oral solid preparation coated with the coating composition of the present invention.

The oral solid preparation comprises, for example, a solid preparation and a coat (coat layer) covering the solid preparation, and the coat at least comprises the coating composition.

Examples of the form of the solid preparation include tablets, granules, fine granules, etc. Among them, tablets are preferred.

The solid preparation may be, for example, a pharmaceutical product, a quasi-pharmaceutical product, a food product, or the like.

Examples of the solid preparation include a pharmaceutical preparation, a quasi-pharmaceutical preparation, a health food (e.g., a food for special dietary uses, a food for specified health uses, a food with nutrient function claims, a functional food, a dietary supplement, a health supplement, a nutritionally fortified food, etc.), etc.

The component of the solid preparation can be appropriately determined based on, for example, the embodiment or application of the solid preparation. The component of the solid preparation may be, for example, an active ingredient, a nutrient, or the like.

The solid preparation usually may comprise an additive that is commonly used in this field (e.g., a filler, a binder, a disintegrant, a lubricant, an antiagglomerant, a solubilizer for pharmaceutical compounds, etc.).

Examples of the filler include saccharides, such as sucrose, lactose, mannitol, and glucose, starch, crystalline cellulose, calcium phosphate, calcium sulfate, etc.

Examples of the binder include PVA, polyacrylic acid, polymethacrylic acid, polyvinyl pyrrolidone, glucose, sucrose, lactose, maltose, dextrin, sorbitol, mannitol, hydroxyethyl cellulose, HPMC, hydroxypropyl cellulose, macrogols, gum arabic, gelatin, agar, starch, etc.

Examples of the disintegrant include low-substituted hydroxypropyl cellulose, carmellose or a salt thereof, croscarmellose sodium, sodium carboxymethyl starch, cross-linked polyvinylpyrrolidone, microcrystalline cellulose, microcrystalline cellulose-carmellose sodium, etc.

Examples of the lubricant or the antiagglomerant include talc, magnesium stearate, calcium stearate, colloidal silica, stearic acid, waxes, hydrogenated oils, polyethylene glycols, sodium benzoate, etc.

Examples of the solubilizer for pharmaceutical compounds include organic acids, such as fumaric acid, succinic acid, malic acid, and adipic acid, etc.

One of these additives or two or more of them may be used.

The amount of the additive can be appropriately determined based on, for example, the kind(s) of the component (s) of the solid preparation.

The oral solid preparation may have a plurality of coat layers.

For example, the oral solid preparation may have an undercoating layer under the coat layer formed from the coating composition of the present invention and may have an overcoating layer over the coat layer.

The undercoating layer and the overcoating layer may be formed by, for example, coating with a composition containing, as a component, a polymer (e.g., HPMC etc.) commonly used for coating of pharmaceutical preparations.

### Method for producing oral solid preparation

The present invention also includes a method for producing an oral solid preparation, which method comprises the step of applying or spraying an aqueous and/or water-based solution containing the coating composition of the present invention to a solid preparation to cover the surface of the solid preparation with the coating composition.

The method for covering the surface of the solid preparation with the coating composition is not particularly limited, and known types of coating, for example, film coating etc. may be used.

The coating technique may be, for example, spray coating.

The coating apparatus used may be, for example, a pan coater, a drum coater, or the like. These apparatuses may be equipped with an air spray, an airless spray, or other types of spray devices.

In one embodiment, the surface of the solid preparation can be covered with the coating composition as follows. A coating composition solution is prepared by dissolving or dispersing the coating composition of the present invention, which optionally contains an additive, in a solvent [e.g., water, an organic solvent (e.g., alcohols such as ethanol etc.), a mixed solvent of water and an organic solvent, etc.], and the solution is sprayed or applied to a solid preparation using the above-mentioned coating apparatus in parallel with drying to cover the surface of the solid preparation.

The coating weight of the coating composition for coating of the surface of the solid preparation varies with the type, form, size, and surface condition of the solid preparation, the properties of the components and additives contained in the solid preparation, and other factors. For example, the coating weight is preferably 1 to 10% by mass, more preferably 1 to 7% by mass, and particularly preferably 2 to 6% by mass relative to the total weight of the solid preparation. When the coating weight is within this range, perfect coating is achieved, and sufficient moisture-proofing, oxygen barrier, and odor masking are provided. In addition, the time for coating is shortened. That is why the range specified above is preferred.

### EXAMPLES

Hereinafter, the present invention will be described in more detail by examples, but the present invention is not limited thereto.

In the following Examples and Comparative Examples, "percentage (%) " and "part" are on a mass basis unless otherwise specified.

### Coating conditions

Apparatus: HICOATER (HC-FZ-Labo, manufactured by Freund Corporation)
Tablet feed: 1000 g
Inlet air temperature: 70 to 80°C
Outlet air temperature: 44 to 52°C
Inlet air flow: 0.6 m³/min
Number of spray guns: 1
Spray gun Spray air flow (atomized air): 30 L/min
Spray gun Spray air flow (patterned air): 9 L/min
Spray rate: adjusted based on the discharge flow from the tube pump
Pan rotation speed: 18 rpm

### Evaluation of coating time

In a coating test, a coating composition solution was initially sprayed at a spray rate of 2.0 g/min, and when no adhesion between tablets or between tablets and the pan occurred, the spray rate was gradually increased until the adhesion between tablets or between tablets and the pan occurred. Once the adhesion occurred, the spray rate was decreased to the extent that no adhesion between tablets or between tablets and the pan occurred. The spray rate was fixed at this level, and spray coating was continued for 10 minutes. When no adhesion occurred during the 10 minutes, this spray rate was regarded as the maximum spray rate. When the adhesion between tablets or between tablets and the pan occurred at the initial spray rate of 2.0 g/min, the spray rate was gradually decreased to the extent that no adhesion between tablets or between tablets and the pan occurred. The spray rate was fixed at this level, and spray coating was continued for 10 minutes. When no adhesion occurred during the 10 minutes, this spray rate was regarded as the maximum spray rate. In addition, the minimum coating time required to achieve a coating mass gain of 3% in terms of the solid content of the tablet was calculated from the maximum spray rate.

### Evaluation of surface smoothness

The surface smoothness of the coated tablets was evaluated in the following conditions.
Measuring instrument: Laser microscope (VK-9510, manufactured by KEYENCE CORPORATION)
Measurement item: Surface roughness: Ra
Measurement magnifications: 50X (objective)

### Evaluation of water vapor transmission rate

A solution or dispersion of the coating composition at a solid content of 10% by mass was cast on a PET sheet, dried in a constant-temperature-and-humidity chamber at 20°C and RH65% to give a 100-µm-thick film. The water vapor transmission rate of the obtained film was measured using an L80-5000 water vapor transmission analyzer (manufactured by Systech Instruments) according to the method specified in JIS K7129 at 25°C and a relative humidity difference of 75%.

### Example 1

12.0 parts by mass of partially saponified PVA (manufactured by JAPAN VAM & POVAL CO., LTD., PE-05JPS, saponification value: 88.2 mol%, viscosity of 4% by mass aqueous solution: 5.3 mPa·s), 3.0 parts by mass of sucrose fatty acid ester (manufactured by Mitsubishi-Chemical Foods Corporation, P-1570, number of carbon atoms per fatty acid ester unit: 16, HLB value: 15), and 15.0 parts by mass of talc (manufactured by Nippon Talc Co., Ltd.) were added to 270.0 parts by mass of purified water with stirring. The mixture was stirred for 1 hour to prepare a coating composition solution (concentration in water: 10% by mass). The coating test was performed using this coating composition solution and a plain tablet composed mainly of lactose and cornstarch to evaluate the maximum spray rate, the minimum coating time, surface roughness, and the water vapor transmission rate of the coating composition.

The maximum spray rate in Example 1 was 6.7 g/min, and the coating time required to achieve a coating mass gain of 3% was 45 minutes. The surface roughness of the tablet was 2.9 µm, and the water vapor transmission rate was 56 g/m²·day. The results are shown in Table 2.

### Examples 2 to 9

The coating test was performed in the same manner as in Example 1 except for using the coating composition consisting of the components described in Table 1 to evaluate the maximum spray rate, the minimum coating time, surface roughness, and the water vapor transmission rate of the coating composition. The results are shown in Table 2.

### Comparative Example 1

13.2 parts by mass of partially saponified PVA (manufactured by JAPAN VAM & POVAL CO., LTD., PE-05JPS, saponification value: 88.2 mol%, viscosity of 4% by mass aqueous solution: 5.3 mPa·s), 3.7 parts by mass of PEG 3000 (manufactured by Wako Pure Chemical Industries, Ltd.), 1.1 parts by mass of lecithin (manufactured by KANTO CHEMICAL CO., INC.), 6.0 parts by mass of talc (manufactured by Nippon Talc Co., Ltd.), and 6.1 parts by mass of titanium oxide (manufactured by Freund Corporation) were added to 270.0 parts by mass of purified water with stirring. The mixture was stirred for 1 hour to prepare a coating composition solution (concentration in water: 10% by mass). The coating test was performed using this coating composition solution and a plain tablet composed mainly of lactose and cornstarch to evaluate the maximum spray rate, the minimum coating time, surface roughness, and the water vapor transmission rate of the coating composition.

The maximum spray rate in Comparative Example 1 was 7.5 g/min, and the coating time required to achieve a coating mass gain of 3% was 40 minutes. The surface roughness was 3.3 µm, and the water vapor transmission rate was 300 g/m²·day. The results are shown in Table 2.

### Comparative Example 2

13.2 parts by mass of partially saponified PVA (manufactured by JAPAN VAM & POVAL CO., LTD., PE-05JPS, saponification value: 88.2 mol%, viscosity of 4% by mass aqueous solution: 5.3 mPa·s), 3.7 parts by mass of PEG 3000 (manufactured by Wako Pure Chemical Industries, Ltd.), 1.1 parts by mass of lecithin (manufactured by KANTO CHEMICAL CO., INC.), 6.0 parts by mass of talc (manufactured by Nippon Talc Co., Ltd.), and 6.1 parts by mass of titanium oxide (manufactured by Freund Corporation) were added to 120.0 parts by mass of purified water with stirring. The mixture was stirred for 1 hour to prepare a coating composition solution (concentration in water: 20% by mass). The coating test was performed using this coating composition solution and a plain tablet composed mainly of lactose and cornstarch to evaluate the maximum spray rate, the minimum coating time, surface roughness, and the water vapor transmission rate of the coating composition.

The maximum spray rate in Comparative Example 2 was 7.2 g/min, and the coating time required to achieve a coating mass gain of 3% was 21 minutes. The surface roughness was 3.8 µm, and the water vapor transmission rate was 300 g/m²·day. The results are shown in Table 2.

### Comparative Example 3

10.5 parts by mass of partially saponified PVA (manufactured by JAPAN VAM & POVAL CO., LTD., PE-05JPS, saponification value: 88.2 mol%, viscosity of 4% by mass aqueous solution: 5.3 mPa·s), 3.3 parts by mass of PEG 3000 (manufactured by Wako Pure Chemical Industries, Ltd.), 7.5 parts by mass of talc (manufactured by Nippon Talc Co., Ltd.), and 8.7 parts by mass of titanium oxide (manufactured by Freund Corporation) were added to 270.0 parts by mass of purified water with stirring. The mixture was stirred for 1 hour to prepare a coating composition solution (concentration in water: 10% by mass). The coating test was performed using this coating composition solution and a plain tablet composed mainly of lactose and cornstarch to evaluate the maximum spray rate, the minimum coating time, surface roughness, and the water vapor transmission rate of the coating composition.

The maximum spray rate in Comparative Example 3 was 7.5 g/min, and the coating time required to achieve a coating mass gain of 3% was 40 minutes. The surface roughness was 3.5 µm, and the water vapor transmission rate was 400 g/m²·day. The results are shown in Table 2.

### Comparative Example 4

30.0 parts by mass of partially saponified PVA (manufactured by JAPAN VAM & POVAL CO. , LTD., PE-05JPS, saponification value: 88.2 mol%, viscosity of 4% by mass aqueous solution: 5.3 mPa·s) was added to 270.0 parts by mass of purified water with stirring. The mixture was stirred for 1 hour to prepare a coating composition solution (concentration in water: 10% by mass). The coating test was performed using this coating composition solution and a plain tablet composed mainly of lactose and cornstarch to evaluate the maximum spray rate, the minimum coating time, surface roughness, and the water vapor transmission rate of the coating composition.

The maximum spray rate in Comparative Example 4 was 3.2 g/min, and the coating time required to achieve a coating mass gain of 3% was 94 minutes. The surface roughness was 3.0 µm, and the water vapor transmission rate was 169 g/m²·day. The results are shown in Table 2.

As is clear in Table 2, Examples 1 to 9, which used the coating composition of the present invention, showed reduced tackiness during coating.

In addition, Examples 1 to 9 showed a low water vapor transmission rate, that is, high moisture-proofness. The water vapor transmission rates in Examples 1 to 9 were all lower than that in Comparative Example 4, which used no plasticizer, indicating that the moisture-proofness was superior to that of conventional coating compositions.

Furthermore, Examples 1 to 9 showed excellent surface smoothness of the coating film.

In contrast, Comparative Examples 1 to 4 showed poor moisture-proofness.

The above results demonstrate that the coating composition of the present invention can provide highly productive and highly moisture-proof coating, etc.

**[Table 1]**

| | | Example 1 | Example 2 | Example 3 | Example 4 | Example 5 | Example 6 | Example 7 | Example 8 | Example 9 | Comparative Example 1 | Comparative Example 2 | Comparative Example 3 | Comparative Example 4 |
|---|---|---|---|---|---|---|---|---|---|---|---|---|---|---|
| | PVA(PE-05JPS) | 40.0 | 40.0 | 40.0 | 30.0 | 45.0 | 66.0 | 40.0 | 40.0 | 40.0 | 44.0 | 44.0 | 35.0 | 100 |
| | HPMC(TC-5R) | 0.0 | 0.0 | 0.0 | 0.0 | 0.0 | 0.0 | 0.0 | 0.0 | 0.0 | 0.0 | 0.0 | 0.0 | 0 |
| | Sucrose fatty acid ester (Number of carbon atoms: 16) | 10.0 | 10.0 | 0.0 | 20.0 | 5.0 | 17.0 | 10.0 | 0.0 | 0.0 | 0.0 | 0.0 | 0.0 | 0 |
| Coating composition | Sucrose fatty acid ester (Number of carbon atoms: 18) | 0.0 | 0.0 | 0.0 | 0.0 | 0.0 | 0.0 | 0.0 | 10.0 | 0.0 | 0.0 | 0.0 | 0.0 | 0.0 |
| | Sucrose fatty acid ester (Number of carbon atoms: 12) | 0.0 | 0.0 | 0.0 | 0.0 | 0.0 | 0.0 | 0.0 | 0.0 | 10.0 | 0.0 | 0.0 | 0.0 | 0.0 |
| | Sorbitan monolaurate | 0.0 | 0.0 | 10.0 | 0.0 | 0.0 | 0.0 | 0.0 | 0.0 | 0.0 | 0.0 | 0.0 | 0.0 | 0.0 |
| | PEG 3000 | 0.0 | 0.0 | 0.0 | 0.0 | 0.0 | 0.0 | 0.0 | 0.0 | 0.0 | 12.4 | 12.4 | 11.0 | 0 |
| | Lecithin | 0.0 | 0.0 | 0.0 | 0.0 | 0.0 | 0.0 | 0.0 | 0.0 | 0.0 | 3.5 | 3.5 | 0.0 | 0 |
| | Talc | 50.0 | 50.0 | 50.0 | 50.0 | 50.0 | 17.0 | 50.0 | 50.0 | 50.0 | 200 | 200 | 25.0 | 0 |
| | Titanium oxide (pigment) | 0.0 | 0.0 | 0.0 | 0.0 | 0.0 | 0.0 | 0.0 | 0.0 | 0.0 | 20.2 | 20.2 | 29.0 | 0.0 |
| | Total | 100.0 | 100.0 | 100.0 | 100.0 | 100.0 | 100.0 | 100.0 | 100.0 | 100.0 | 100.0 | 100.0 | 100.0 | 100.0 |
| | Aqueous solution conc. (%) | 10 | 20 | 10 | 10 | 10 | 10 | 10 | 10 | 10 | 10 | 20 | 10 | 10 |

**[Table 2]**

| | | Example 1 | Example 2 | Example 3 | Example 4 | Example 5 | Example 6 | Example 7 | Example 8 | Example 9 | Comparative Example 1 | Comparative Example 2 | Comparative Example 3 | Comparative Example 4 |
|---|---|---|---|---|---|---|---|---|---|---|---|---|---|---|
| Coating results | Maximum spray rate (g/min.) | 6.7 | 5.5 | 7.0 | 7.5 | 7.5 | 7.5 | 7.0 | 6.7 | 5.5 | 7.5 | 7.2 | 7.5 | 3.2 |
| | Coating time (min.) | 45 | 27 | 43 | 40 | 40 | 40 | 43 | 45 | 55 | 40 | 21 | 40 | 94 |
| | Surface roughness (µm) | 2.9 | 3.2 | 2.9 | 2.9 | 2.9 | 3.3 | 3.0 | 3.0 | 3.0 | 3.3 | 3.8 | 3.5 | 3.0 |
| Film property | Water vapor transmission rate (g/m²·day) | 56 | 56 | 50 | 49 | 57 | 90 | 58 | 54 | 70 | 300 | 300 | 400 | 169 |

### INDUSTRIAL APPLICABILITY

The coating composition of the present invention can provide fast and highly productive coating and form a coating film having excellent moisture-proofness. Therefore, the coating composition of the present invention is very useful industrially in the production of oral solid preparations.

## Claims

1. A coating composition for oral solid preparations, the coating composition comprising a polyvinyl alcohol-based polymer (a), a fatty acid ester of a polyol having 3 or more hydroxy groups (b), and talc (c),
wherein the coating composition meets the following requirements (1) to (4):
(1) the fatty acid ester of a polyol having 3 or more hydroxy groups (b) has a hydrophilic lipophilic balance (HLB) value of 4.0 or more;
(2) the fatty acid ester of a polyol having 3 or more hydroxy groups (b) has 12 to 22 carbon atoms per fatty acid ester unit;
(3) the talc (c) is present at 50% by mass or less of the whole composition;
and
(4) the proportion of the polyol fatty acid ester (b) in the whole composition is 5% by mass or more.

2. The coating composition for oral solid preparations according to claim 1, wherein the fatty acid ester of a polyol having 3 or more hydroxy groups (b) comprises at least one kind selected from a sugar fatty acid ester and a sugar alcohol fatty acid ester.

3. The coating composition for oral solid preparations according to claim 1, wherein the fatty acid ester of a polyol having 3 or more hydroxy groups (b) comprises a sorbitan fatty acid ester.

4. The coating composition for oral solid preparations according to any one of claims 1 to 3, wherein the polyvinyl alcohol-based polymer (a) and the fatty acid ester of a polyol having 3 or more hydroxy groups (b) are present at a mass ratio of 95:5 to 50:50 in the coating composition.

5. The coating composition for oral solid preparations according to any one of claims 1 to 4, the coating composition further comprising a colorant.

6. The coating composition for oral solid preparations according to any one of claims 1 to 5, wherein a 4% by mass aqueous solution of the polyvinyl alcohol-based polymer (a) has a viscosity of 2.0 to 10.0 mPa·s.

7. An oral solid preparation coated with the coating composition according to any one of claims 1 to 6.

8. A method for producing an oral solid preparation, the method comprising the step of applying or spraying an aqueous and/or water-based solution containing the coating composition according to any one of claims 1 to 6 to a solid preparation to cover a surface of the solid preparation with the coating composition.

9. Use of a composition for coating an oral solid preparation, the composition comprising a polyvinyl alcohol-based polymer (a), a fatty acid ester of a polyol having 3 or more hydroxy groups (b), and talc (c),
wherein the composition meets the following requirements (1) to (4) :
(1) the fatty acid ester of a polyol having 3 or more hydroxy groups (b) has a hydrophilic lipophilic balance (HLB) value of 4.0 or more;
(2) the fatty acid ester of a polyol having 3 or more hydroxy groups (b) has 12 to 22 carbon atoms per fatty acid ester unit;
(3) the talc (c) is present at 50% by mass or less of the whole composition;
and
(4) the proportion of the polyol fatty acid ester (b) in the whole composition is 5% by mass or more.

## Patentansprüche

1. Beschichtungszusammensetzung für orale feste Präparate, wobei die Beschichtungszusammensetzung ein Polyvinylalkohol-basiertes Polymer (a), einen Fettsäureester eines Polyols mit 3 oder mehr Hydroxygruppen (b) und Talk (c) umfasst, wobei die Beschichtungszusammensetzung die folgenden Anforderungen (1) bis (4) erfüllt:
(1) der Fettsäureester eines Polyols mit 3 oder mehr Hydroxygruppen (b) hat einen Hydrophil-lipophil-Gleichgewichts (HLB)-Wert von 4,0 oder mehr;
(2) der Fettsäureester eines Polyols mit 3 oder mehr Hydroxygruppen (b) weist 12 bis 22 Kohlenstoffatome pro Fettsäureestereinheit auf;
(3) der Talk (c) liegt mit 50 Masse-% oder weniger der gesamten Zusammensetzung vor; und
(4) der Anteil des Polyolfettsäureesters (b) in der gesamten Zusammensetzung beträgt 5 Masse-% oder mehr.

2. Beschichtungszusammensetzung für orale feste Präparate nach Anspruch 1, wobei der Fettsäureester eines Polyols mit 3 oder mehr Hydroxygruppen (b) mindestens eine Art, ausgewählt aus einem Zuckerfettsäureester und einem Zuckeralkoholfettsäureester, umfasst.

3. Beschichtungszusammensetzung für orale feste Präparate nach Anspruch 1, wobei der Fettsäureester eines Polyols mit 3 oder mehr Hydroxygruppen (b) einen Sorbitanfettsäureester umfasst.

4. Beschichtungszusammensetzung für orale feste Präparate nach einem der Ansprüche 1 bis 3, wobei das Polyvinylalkohol-basierte Polymer (a) und der Fettsäureester eines Polyols mit 3 oder mehr Hydroxygruppen (b) in einem Masseverhältnis von 95:5 bis 50:50 in der Beschichtungszusammensetzung vorliegen.

5. Beschichtungszusammensetzung für orale feste Präparate nach einem der Ansprüche 1 bis 4, wobei die Beschichtungszusammensetzung ferner ein Färbemittel umfasst.

6. Beschichtungszusammensetzung für orale feste Präparate nach einem der Ansprüche 1 bis 5, wobei eine wässerige Lösung von dem Polyvinylalkohol-basierten Polymer (a) von 4 Masse-% eine Viskosität von 2,0 bis 10,0 mPa·s aufweist.

7. Orales festes Präparat, beschichtet mit der Beschichtungszusammensetzung nach einem der Ansprüche 1 bis 6.

8. Verfahren zur Herstellung eines oralen festen Präparats, wobei das Verfahren den Schritt des Aufbringens oder Sprühens einer wässerigen und/oder Wasser-basierten Lösung, die die Beschichtungszusammensetzung nach einem der Ansprüche 1 bis 6 enthält, auf ein festes Präparat, um eine Oberfläche des festen Präparats mit der Beschichtungszusammensetzung zu bedecken, umfasst.

9. Verwendung einer Zusammensetzung zum Beschichten eines oralen festen Präparats, wobei die Zusammensetzung ein Polyvinylalkohol-basiertes Polymer (a), einen Fettsäureester eines Polyols mit 3 oder mehr Hydroxygruppen (b) und Talk (c) umfasst, wobei die Beschichtungszusammensetzung die folgenden Anforderungen (1) bis (4) erfüllt:
(1) der Fettsäureester eines Polyols mit 3 oder mehr Hydroxygruppen (b) hat einen Hydrophil-lipophil-Gleichgewichts- (HLB)-Wert von 4,0 oder mehr;
(2) der Fettsäureester eines Polyols mit 3 oder mehr Hydroxygruppen (b) weist 12 bis 22 Kohlenstoffatome pro Fettsäureestereinheit auf;
(3) der Talk (c) liegt mit 50 Masse-% oder weniger der gesamten Zusammensetzung vor; und
(4) der Anteil des Polyolfettsäureesters (b) in der gesamten Zusammensetzung beträgt 5 Masse-% oder mehr.

## Revendications

1. Composition d'enrobage pour préparations solides destinées à être administrées par voie orale, la composition d'enrobage comprenant un polymère à base d'alcool polyvinylique (a), un ester d'acide gras d'un polyol ayant 3 groupes hydroxy ou plus (b), et du talc (c),
dans laquelle la composition d'enrobage répond aux exigences suivantes (1) à (4) :
(1) l'ester d'acide gras d'un polyol ayant 3 groupes hydroxy ou plus (b) a une valeur d'équilibre hydrophile-lipophile (HLB) égale ou supérieure à 4,0 ;
(2) l'ester d'acide gras d'un polyol ayant 3 groupes hydroxy ou plus (b) a 12 à 22 atomes de carbone par unité d'ester d'acide gras ;
(3) le talc (c) est présent à hauteur de 50 % en masse ou moins de l'ensemble de la composition ;
et
(4) la proportion de l'ester d'acide gras de polyol (b) dans l'ensemble de la composition est égale ou supérieure à 5 % en masse.

2. Composition d'enrobage pour préparations solides destinées à être administrées par voie orale selon la revendication 1, dans laquelle l'ester d'acide gras d'un polyol ayant 3 groupes hydroxy ou plus (b) comprend au moins un type choisi parmi un ester d'acide gras de sucre et un ester d'acide gras d'alcool de sucre.

3. Composition d'enrobage pour préparations solides destinées à être administrées par voie orale selon la revendication 1, dans laquelle l'ester d'acide gras d'un polyol ayant 3 groupes hydroxy ou plus (b) comprend un ester d'acide gras de sorbitane.

4. Composition d'enrobage pour préparations solides destinées à être administrées par voie orale selon l'une quelconque des revendications 1 à 3, dans laquelle le polymère à base d'alcool polyvinylique (a) et l'ester d'acide gras d'un polyol ayant 3 groupes hydroxy ou plus (b) sont présents dans un rapport massique de 95:5 à 50:50 dans la composition d'enrobage.

5. Composition d'enrobage pour préparations solides destinées à être administrées par voie orale selon l'une quelconque des revendications 1 à 4, la composition d'enrobage comprenant en outre un colorant.

6. Composition d'enrobage pour préparations solides destinées à être administrées par voie orale selon l'une quelconque des revendications 1 à 5, dans laquelle une solution aqueuse à 4 % en masse du polymère à base d'alcool polyvinylique (a) a une viscosité de 2,0 à 10,0 mPa·s.

7. Préparation solide destinée à être administrée par voie orale enrobée de la composition d'enrobage selon l'une quelconque des revendications 1 à 6.

8. Procédé de fabrication d'une préparation solide destinée à être administrée par voie orale, le procédé comprenant l'étape d'application ou de pulvérisation d'une solution aqueuse et/ou à base d'eau contenant la composition d'enrobage selon l'une quelconque des revendications 1 à 6 sur une préparation solide afin de couvrir une surface de la préparation solide avec la composition d'enrobage.

9. Utilisation d'une composition pour l'enrobage d'une préparation solide destinée à être administrée par voie orale, la composition comprenant un polymère à base d'alcool polyvinylique (a), un ester d'acide gras d'un polyol ayant 3 groupes hydroxy ou plus (b), et le talc (c),
dans laquelle la composition répond aux exigences suivantes (1) à (4) :
(1) l'ester d'acide gras d'un polyol ayant 3 groupes hydroxy ou plus (b) a une valeur d'équilibre hydrophile-lipophile (HLB) de 4,0 ou plus ;
(2) l'ester d'acide gras d'un polyol ayant 3 groupes hydroxy ou plus (b) a 12 à 22 atomes de carbone par unité d'ester d'acide gras ;
(3) le talc (c) est présent à hauteur de 50 % en masse ou moins de l'ensemble de la composition ;
et
(4) la proportion de l'ester d'acide gras de polyol (b) dans l'ensemble de la composition est égale ou supérieure à 5 % en masse.
